# EUROPÄISCHE PATENTANMELDUNG

(11) **EP 1 738 788 A1**
(43) Veröffentlichungstag der Anmeldung: **03.01.2007**
(21) Anmeldenummer: 06013208.1
(22) Anmeldetag: 27.06.2006
(51) Int. Cl.: A61M 16/04

(54) **Trachealkanüle mit drehbarem Konnektor**

(30) Priorität: 27.06.2005 DE 102005030302
(71) Anmelder: PRIMED HALBERSTADT MEDIZINTECHNIK GMBH, 38820 Halberstadt (DE)
(72) Erfinder: Klimenta, Klaus, 38835 Zilly (DE); Leibitzki, Harry, 38889 Blankenburg (DE)
(74) Vertreter: Bock, Gerhard

(57) **Zusammenfassung**

Die Erfindung betrifft eine Trachealkanüle mit Konnektor.

Die Aufgabe der Erfindung, eine Trachealkanüle, bestehend aus Außen- und Innenrohr, mit Konnektor anzugeben, die die Nachteile des Standes der Technik vermeidet und eine leicht ausführbare Rotationsbewegung von einem Bauteil, das durch den Konnektor gehaltert wird, und dem Außen- oder Innenrohr zueinander ermöglicht, wird dadurch gelöst, dass eine Trachealkanüle aus einem Innen- und einem Außenrohr besteht, wobei das distale Ende des Innenrohrs mit einem Konnektor verbunden und der Konnektor drehbar ist.

## Beschreibung

Die Erfindung betrifft eine Trachealkanüle mit Konnektor.

Verschiedenste Bauformen von Trachealkanülen (Tracheostomaprothesen) sind seit Jahrzehnten bekannt.

Die Schrift DE 101 09 935.5-09 offenbart bspw. eine Tracheostomaprothese, die aus einem Sprechventil mit einer Klappe, einer Außenkanüle, die mit einem sie ringförmig umgebenden Dichtballon , der über einen Luftkanal, welcher innerhalb der Außenkanüle verläuft, mit einem Druckballon verbunden ist, und die mit Perforierungen, die sich zwischen dem Dichtballon und dem Sprechventil nahe dem Dichtballon befinden, versehen ist, einer Innenkanüle mit einer Ausnehmung, die sich gegenüber den Perforierungen versetzt, nahe dem Sprechventil befindet, einer Dichtung , die zwischen der Innenkanüle und der Außenkanüle nahe dem Dichtballon dichtend angeordnet ist, und einer Absaugeinrichtung, die mit einem Absaugkanal oberhalb des Sprechventils verbunden ist, besteht.

Aus der Schrift DE 195 14 433 C1 ist eine Tracheostomiekanüle zum Einsatz in ein Tracheostoma bekannt, welche aus einer schlauchförmigen Außenkanüle mit Kanülenschild und einer schlauchförmigen Innenkanüle besteht, wobei die Innenkanüle in die Außenkanüle einführbar und mit dieser am proximalen Teil verriegelbar ist.

Aus der Schrift DE 196 36 050.1 ist ein Tracheostomaplatzhalter bekannt, der aus einem rohrförmigen Tracheostomaschenkel, der einseitig in einen einstückig angeformten und in einer ersten Dimension (y) gewölbt ausgebildeten Trachealschenkel ausläuft, wobei der Trachealschenkel in einer zur ersten Dimension senkrecht verlaufenden Dimension (z) amittig zur Tracheostomaschenkelachse (X-X) derart angebunden ist, dass er unterschiedlich lange Überhangbereiche aufweist und dem Tracheostomaschenkel Rastmittel zugeordnet sind, die die Aufnahme und Fixierung eines Schildes in einem variabel anpassbaren Abstand zur Anlagefläche des Trachealschenkels an die Trachea gewährleisten.
Dabei sind die Rastmittel in Form von in den Tracheostomaschenkel eingebrachten Nuten bzw. in Form von auf dem Tracheostomaschenkel aufgebrachten Wulsten ausgebildet.

Die Schrift DE 20 2004 007 251 U1 offenbart eine Trachealkanüle bestehend aus einer Innenkanüle und einer Außenkanüle, wobei die Innenkanüle an ihrem distalen Ende n Rastmittel aufweist, die nicht durchgehend kreisförmig den Querschnitt des distalen Endes des Innenrohrs ausbilden, sondern zapfen- oder widerhakenartig aus dem distalen Ende des Innenrohrs herausragen und formschlüssig in entsprechende Ausnehmungen / Profile der Außenkanüle greifen, wobei n ≥ 3 ist, so dass ein Verdrehen der Innenkanüle zur Außenkanüle verhindert wird.

Die Schrift DE 20 2004 007 252 U1 offenbart Trachealkanüle bestehend aus einer Innenkanüle und einer Außenkanüle, wobei die Innenkanüle über Rastmittel auf ihrer Außenwand verfügt, die der Innenkanüle einen n-eckigen Querschnitt verleihen, wobei n ≥ 3 ist, und die formschlüssig in die Innenwand der Außenkanüle greifen, so dass ein Verdrehen der Innenkanüle zur Außenkanüle verhindert wird.

Aus der Schrift DE 35 23 663 A1 ist eine Endotrachealkanüle (-tubus) bekannt, an deren einem Ende ein Anschluss für ein Beatmungsgerät und an deren anderem Ende eine Öffnung für den Ein- bzw. Austritt des Beatmungsmediums vorgesehen ist.

Aus der Schrift DE 20 2004 013 323 U1 ist ein TracheostomaPlatzhalter, bestehend aus einem rohrförmigen Tracheostoma-Schenkel, der einseitig aus einem Stück mit einem angespritzten und gewölbt ausgebildeten Tracheal-Schenkel ausgebildet ist und einem auf dem Tracheostoma-Schenkel angespritzten, angeklebten oder verschiebbaren sowie arretierbaren Flansch mit einer mehrteiligen Klemmhülse für formschlüssige oder stoffschlüssige Verbindung mit einem Aufnahmering zur Montage von Sprechventilen und künstlichen Nasen, bekannt.

Der Nachteil aller bekannten Tracheostomaprothesen / Trachealkanülen ist, dass die Konnektoren bzw. Haltemittel (soweit vorhanden), die für die Montage von Bauteilen, wie bspw. Beatmungszubehör, Filtern, Sprechventilen oder künstlichen Nasen, form- und stoffschlüssig ausgeführt sind, eine starre Verbindung dieser Bauteile zu den Tracheostomaprothesen / Trachealkanülen mit sich bringen, so dass beim Gebrauch dieser keine leicht ausführbare Rotationsbewegungen von Bauteil und Tracheostomaprothese / Trachealkanüle zueinander erfolgen kann. In Folge dessen kann gemäß dem Stand der Technik kein, für die Verwendung wünschenswerter Druck-/Zugausgleich an den Tracheostomaprothesen mit Konnektoren (Trachealkanülen mit Haltemittel) durch eine leicht ausführbare Rotationsbewegung erfolgen. Darüber hinaus ist bei den bekannten Trachealkanülen nachteilig, dass die Luftdurchtrittsöffnung durch die Konnektoren am Außenrohr erheblich eingeschränkt ist, was zu einem erhöhten Atemwiderstand bzw. zu größeren Durchmessern der Trachealkanülen führt.
Außerdem besitzen diese Kanülen kein austauschbares Innenrohr, so dass eine Reinigung / Desinfektion nur durch einen kompletten Kanülenwechsel möglich ist.

Der Erfindung liegt daher die Aufgabe zugrunde, eine Trachealkanüle, bestehend aus Außen- und Innenrohr, mit Konnektor anzugeben, die die Nachteile des Standes der Technik vermeidet und eine leicht ausführbare Rotationsbewegung von einem Bauteil, das durch den Konnektor gehaltert wird, und dem Außen- oder Innenrohr zueinander ermöglicht.

Gemäß der Erfindung wird diese Aufgabe durch die kennzeichnenden Merkmale des ersten Patentanspruchs gelöst und durch vorteilhafte Ausgestaltungen gemäß den Unteransprüchen ergänzt.

Das Wesen der Erfindung besteht darin, dass die Trachealkanüle aus einem Innen- und einem Außenrohr aufgebaut ist, wobei das distale Ende des Innenrohrs einen Konnektor aufweist, der frei um die Achse X-X drehbar ist.
Dadurch kann das Innenrohr mit Standartaußenrohren (geblockt oder ungeblockt) zu einer kompletten Trachealkanüle zusammengesetzt werden, wobei der Konnektor mit verschieden Zubehörteilen, wie bspw. Beatmungsschlauch bzw. Sprechventil versehen werden kann. Dies hat gegenüber den bisher bekannten Lösungen den Vorteil, dass das Innenrohr komplett mit Zubehörteilen aus der Außenkanüle entnommen werden kann, bspw. zum Reinigen / Desinfizieren bzw. zum Anschluss an ein Beatmungsgerät, und dabei das Außenrohr im Patienten verbleiben kann.

Die drehbare Verbindung zwischen dem Innenrohr und dem Konnektor wird durch einen Flansch am distalen Ende des Innenrohrs und einen Flansch an der, diesem distalen Ende zugewandten Seite des Konnektors, die miteinander vermittels eines inneren und eines äußeren Haltemittels formschlüssig verbunden sind, gebildet, wobei der Flansch des Innenrohrs gleichzeitig das innere Haltemittel bildet.
Innenrohr und Flansch können sowohl ein- oder auch zweistückig ausgeführt sein.

Der Flansch des Konnektors ist mit dem Flansch des Innenrohrs durch das innere und das äußere Haltemittel in der Art verbunden, dass zwischen Konnektor und Rohr ein ringförmiger Zwischenraum gebildet ist, wobei das äußere Haltemittel annähernd zentral eine Ausnehmung aufweist, durch die der Konnektor führt, wobei der Flansch des Konnektors einen Anschlag gegenüber dem äußeren Haltemittel bildet, so dass der Konnektor nicht durch die Ausnehmung des äußeren Haltemittels rutschen kann.

Das innere und das äußere Haltemittel sind miteinander form- und stoffschlüssig verbunden. Diese Verbindung kann bspw. durch Anspritzen, Verkleben oder Verschweißen erfolgen.

Vorteilhafter Weise ist der Konnektor gegenüber dem Innenrohr in dem ringförmigen Zwischenraum gelagert, so dass der Konnektor gegenüber dem Rohr besonders leichtgängig drehbar ist.

Die Lagerung kann bspw. durch kugelförmige Lagerkörper, die im ringförmigen Zwischenraum zwischen dem distalen Ende des Rohrs und der, diesem Ende zugewandten Seite des Konnektors platziert sind, ausgebildet sein.
Dabei können die Lagerkörper ein- oder zweistückig mit dem Rohr oder dem Konnektor stoffschlüssig verbunden ausgeführt sein bzw. sich frei beweglich zwischen dem Rohr und dem Konnektor befinden.

Alle Bauteile der erfindungsgemäßen Innenkanüle mit Konnektor können bspw. aus Kunststoff / Plastik und/ oder Metall gefertigt sein.

Durch die Erfindung wird ein leicht ausführbares Verdrehen des Konnektors gegenüber der Innenkanüle ermöglicht, so dass ein Druck-/ Zugausgleich an der Trachealkanüle mit Konnektor bei benutzungsbediengten Bewegungen des durch den Konnektor gehalterten Bauteils bewirkt wird.
Außerdem ist ein Entnehmen der Innenkanüle mit dazugehörigen Zusatzteilen möglich, ohne dass die Außenkanüle entnommen werden muss.

Die Erfindung wird nachstehend an Hand der schematischen Zeichnung näher erläutert. Es zeigt:

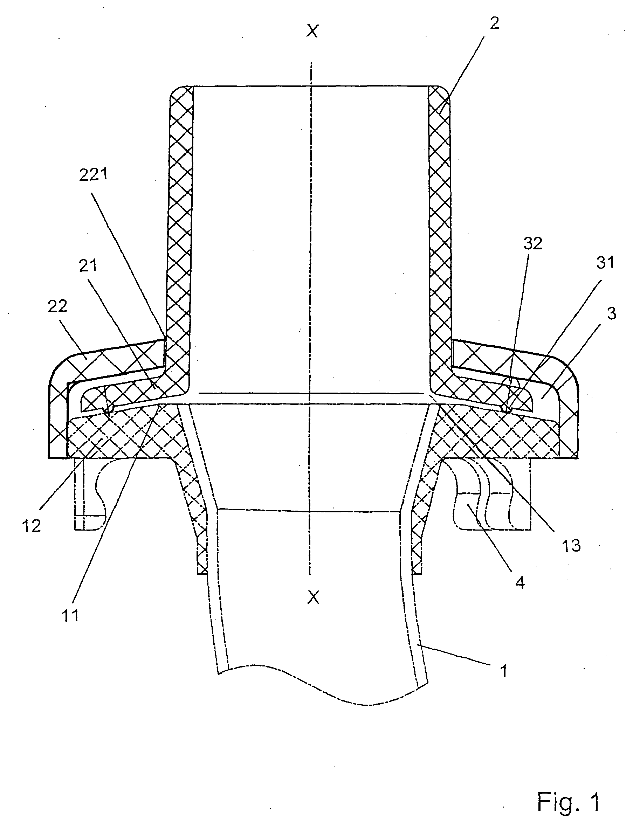

- Fig. 1:: eine Ausführungsform eines Innenrohrs der erfindungsgemäßen Trachealkanüle mit Konnektor im Querschnitt.

Das in Fig. 1 dargestellte Innenrohr (1) der erfindungsgemäßen Trachealkanüle weist an seinem distalen Ende (13) einen Konnektor (2) auf, der frei um die Achse X-X drehbar ist.

Die drehbare Verbindung zwischen dem Innenrohr (1) und dem Konnektor (2) wird durch einen Flansch (11) am distalen Ende (13) des Innenrohrs (1) und einen Flansch (21) an der, diesem distalen Ende (13) zugewandten Seite des Konnektors (2), die miteinander vermittels eines inneren und eines äußeren Haltemittels (12 und 22) formschlüssig verbunden sind, gebildet, wobei der Flansch (12) des Innenrohrs (1) gleichzeitig das innere Haltemittel (12) bildet.
Innenrohr (1) und Flansch (12) können sowohl ein- oder auch zweistückig ausgeführt sein.

Der Flansch (21) des Konnektors (4) ist mit dem Flansch (11) des Innenrohrs (1) durch das innere und das äußere Haltemittel (12 und 22) in der Art verbunden, dass zwischen Konnektor (2) und Innenrohr (1)ein ringförmiger Zwischenraum (3) gebildet ist, wobei das äußere Haltemittel 22) annähernd zentral eine Ausnehmung (221) aufweist, durch die der Konnektor (2) führt, wobei der Flansch (21) des Konnektors (2) einen Anschlag gegenüber dem äußeren Haltemitte (22) bildet, so dass der Konnektor (2) nicht durch die Ausnehmung (221) des äußeren Haltemittels (22) rutschen kann.

Das innere und das äußere Haltemittel (12 und 22) sind miteinander form- und stoffschlüssig verbunden. Diese Verbindung (32) kann bspw. durch Verkleben, Verschweißen oder Anspritzen erfolgen.

Vorteilhafter Weise ist der Konnektor (2) gegenüber dem Innenrohr (1) in dem ringförmigen Zwischenraum (3) gelagert, so dass der Konnektor (2) gegenüber dem Innenrohr (1) besonders leichtgängig drehbar ist.

Die Lagerung kann bspw. durch kugelförmige Lagerkörper (31), die im ringförmigen Zwischenraum (3) zwischen dem distalen Ende (13) des Innenrohrs (1) und der, diesem Ende zugewandten Seite des Konnektors (2) platziert sind, ausgebildet sein.
Dabei können die Lagerkörper (31) ein- oder zweistückig mit dem Innenrohr (1) oder dem Konnektor (2) stoffschlüssig verbunden ausgeführt sein bzw. sich frei beweglich zwischen dem Rohr und dem Konnektor befinden.

Das innere Haltemittel (12) kann an seiner, dem Flansch (21) abgewandten Seite mit einer Verriegelung (4) versehen sein, die der Verbindung des Innenrohrs (1) mit dem Außenrohr der Trachealkanüle dient.

Alle Bauteile der erfindungsgemäßen Trachealkanüle mit Konnektor können bspw. aus Kunststoff / Plastik und/ oder Metall gefertigt sein.

Durch die Erfindung wird ein leicht ausführbares Verdrehen des Konnektors gegenüber der Innenkanüle ermöglicht, so dass ein Druck-/ Zugausgleich an der Trachealkanüle mit Konnektor bei benutzungsbediengten Bewegungen des durch den Konnektor gehalterten Bauteils bewirkt wird. Gleichzeitig ist ein Entnehmen des Innenrohrs (1) komplett mit den Zusatzteilen, wie bspw. Anschluss eines Beatmungsgeräts oder Sprechventil, möglich ohne dass das Außenrohr aus dem Patienten entnommen werden muss.

Alle in der Beschreibung, den nachfolgenden Ansprüchen und der Zeichnung dargestellten Merkmale können sowohl einzeln als auch in beliebiger Kombination miteinander erfindungswesentlich sein.

### Bezugszeichenliste

- 1 -: Innenrohr
- 11 -: Flansch
- 12 -: inneres Haltemittel
- 13 -: distales Ende
- 2 -: Konnektor
- 21 -: Flansch
- 22 -: Haltemittel
- 221 -: Ausnehmung
- 3 -: Zwischenraum
- 31 -: Lagerkörper
- 32 -: Verbindung
- 4 -: Verriegelung
- X-X -: Achse

## Patentansprüche

1. Trachealkanüle bestehend aus einem Innen- und einem Außenrohr, **dadurch gekennzeichnet, dass** das distale Ende (13) des Innenrohrs (1) mit einem Konnektor (2) verbunden und der Konnektor (2) drehbar ist.

2. Trachealkanüle gemäß Anspruch 1, **dadurch gekennzeichnet, dass** die drehbare Verbindung zwischen dem Innenrohr (1) und dem Konnektor (2) durch einen Flansch (21') am distalen Ende (13) des Innenrohrs (1) und einen Flansch (21) an der, diesem distalen Ende (13) zugewandten Seite des Konnektors (2), die miteinander vermittels eines inneren und eines äußeren Haltemittels (22) formschlüssig verbunden sind, gebildet wird, wobei der Flansch (21') des Innenrohrs (1) gleichzeitig das innere Haltemittel (22) bildet.

3. Trachealkanüle gemäß Anspruch 2, **dadurch gekennzeichnet, dass** Innenrohr (1) und Flansch (21') ein- oder zweistückig ausgebildet sind.

4. Trachealkanüle gemäß Anspruch 2, **dadurch gekennzeichnet, dass** der Flansch (21) des Konnektors (2) mit dem Flansch (21') des Innenrohrs (1) durch das innere und das äußere Haltemittel (22) in der Art verbunden ist, dass zwischen Konnektor (2) und Innenrohr (1) ein ringförmiger Zwischenraum (3) gebildet ist, wobei das äußere Haltemittel (22) annähernd zentral eine Ausnehmung (221) aufweist, durch die der Konnektor (2) führt, wobei der Flansch (22) des Konnektors (2) einen Anschlag gegenüber dem äußeren Haltemittel (22) bildet, so dass der Konnektor (2) in der Ausnehmung (221) des äußeren Haltemittels (22) gehaltert wird.

5. Trachealkanüle gemäß Anspruch 2, **dadurch gekennzeichnet, dass** das innere und das äußere Haltemittel (22) miteinander form- und stoffschlüssig verbunden sind.

6. Trachealkanüle gemäß Anspruch 5, **dadurch gekennzeichnet, dass** die Verbindung durch Anspritzen, Verkleben oder Verschweißen gebildet ist.

7. Trachealkanüle gemäß Anspruch 2, **dadurch gekennzeichnet, dass** der Konnektor (2) gegenüber Innenrohr(1) in dem ringförmigen Zwischenraum (3) gelagert ist, so dass der Konnektor (2) gegenüber dem Innenrohr (1) besonders leichtgängig drehbar ist.

8. Trachealkanüle gemäß Anspruch 7, **dadurch gekennzeichnet, dass** die Lagerung durch kugelförmige Lagerkörper (31), die im ringförmigen Zwischenraum (3) zwischen dem distalen Ende (13) des Innenrohrs (1) und der, diesem Ende (13) zugewanden Seite des Konnektors (2) platziert sind, gebildet ist.

9. Trachealkanüle gemäß Anspruch 8, **dadurch gekennzeichnet, dass** die Lagerkörper (31) ein- oder zweistückig mit dem Innenrohr (1) oder dem Konnektor (2) stoffschlüssig verbunden ausgeführt sind.

10. Trachealkanüle gemäß Anspruch 8, **dadurch gekennzeichnet, dass** sich die Lagerkörper (31) frei beweglich zwischen dem Innenrohr (1) und dem Konnektor (2) befinden.

11. Trachealkanüle nach einem oder mehreren der voran stehenden Schutzansprüche, **dadurch gekennzeichnet, dass** das Innenrohr (1) mit dem Außenrohr über eine Verriegelung (4) verbindbar ist.

12. Trachealkanüle nach einem oder mehreren der voran stehenden Schutzansprüche, **dadurch gekennzeichnet, dass** alle Bauteile aus Kunststoff und / oder Metall bestehen.
